# EUROPEAN PATENT APPLICATION

(11) **EP 3 628 651 A1**
(43) Date of publication of application: **01.04.2020**
(21) Application number: 19382805.0
(22) Date of filing: 17.09.2019
(51) Int. Cl.: C05F 11/00, C02F 3/32

(54) **METHOD FOR OBTAINING CONCENTRATES OF BIOFERTILIZERS AND BIOSTIMULANTS FOR AGRICULTURAL USE FROM BIOMASS OF MICROALGAE, INCLUDING CYANOBACTERIA**

(30) Priority: 25.09.2018 ES 201830923
(71) Applicant: Biorizon Biotech, S.L., 04131 Almería (ES)
(72) Inventor: Rojas Crespo, Elisa, 04131 Almería (ES); Iglesias Hernández, David, 04131 Almería (ES); Acien Fernández, Francisco Gabriel, 04131 Almería (ES); Pozo Dengra, Joaquin, 04131 Almería (ES)
(74) Representative: Capitán García, Maria Nuria

(57) **Abstract**

A method for obtaining biofertilizer concentrates and biostimulants for agricultural use from biomass of microalgae, including cyanobacteria, wherein, firstly, the cellular content of the microalgae, including cyanobacteria, is released by rupturing the cells thereof; next, the protein fraction and the cell wall of the microalgae, including cyanobacteria, obtained from said release of the cellular content, are hydrolyzed with proteolytic enzymes until producing free amino acids; next, nitrogen (N), phosphorus (P) and/or potassium (K) excipients are added to the previously obtained product of enzymatic hydrolysis.

## Description

### TECHNICAL FIELD OF THE INVENTION

The present invention belongs to the field of producing fertilizing and biostimulant products for agricultural use.

Said invention relates to a method which allows the production of a fertilizing and biostimulant product for agricultural use, rich in free amino acids, peptides and naturally occurring phytohormones from microalgae, including cyanobacteria, applicable both in extensive and intensive farming.

### BACKGROUND OF THE INVENTION

As is known, the excessive use of agrochemicals of synthetic origin has led to global environmental degradation, causing the emergence of dead zones in oceans, eutrophication, soil infertility and loss of biodiversity.

As an alternative to the environmental disaster caused by the excessive use of synthetic fertilizers, there are biofertilizers, which constitute a cost-effective and sustainable alternative to these synthetic fertilizers, since they not only improve agricultural production, but their use additionally reduces environmental pollution.

Biofertilizer products are defined as those containing live microorganisms or natural compounds derived from organisms such as bacteria, fungi and algae that improve the chemical and biological properties of the soil, stimulate plant growth and restore soil fertility.

Biofertilizer products based on microalgae, including cyanobacteria, are known. Microalgae, including cyanobacteria, contain high levels of micronutrients and macronutrients essential for plant growth, hence their application in the production of biofertilizers. The association between greater nutrient absorption, greater biomass accumulation and higher crop yields and the incorporation of biofertilizers of microalgae, including cyanobacteria, has been demonstrated. It has been confirmed that cell extracts and the growth medium of several species of microalgae, including cyanobacteria, contain p0hytohormones (gibberellins, auxins and cytokinins), which have been shown to play a crucial role in plant development.

For the production of microalgae, including cyanobacteria, clean water and chemical fertilizers can be used; however, the biotreatment of wastewater with microalgae, including cyanobacteria, is particularly interesting due to their photosynthetic capabilities, converting solar energy into useful biomass and incorporating nutrients such as nitrogen (N), phosphorus (P) and/or potassium (K) that cause eutrophication.

The use of microalgae, including cyanobacteria, has been reported in the treatment of human wastewater, livestock waste, agro-industrial waste and industrial effluents. Similarly, microalgae, including cyanobacteria, have proven to be effective in the treatment of waste such as slurry, effluent from food-processing factories and other agricultural waste. Microalgae, including cyanobacteria, have also been used in the elimination of toxic minerals such as lead (Pb), cadmium (Cd), mercury (Hg), scandium (Sc), tin (Sn), arsenic (As) and bromine (Br).

Microalgae, including cyanobacteria, cultured in wastewater treatment systems or wastewater effluents constitute potentially important biomass for various applications, such as biofuels, extraction of value-added compounds, biofertilizers, etc. However, most wastewater treatment systems that use biomass of microalgae, including cyanobacteria, do not include the harvesting thereof. Normally, the biomass is discharged into ponds, where it decomposes at the bottom of the pond, releasing methane into the atmosphere and degrading water quality. Instead, the biomass of microalgae, including cyanobacteria, could be processed, for example, to produce biofertilizers and biostimulants for agriculture.

For this reason, it is necessary to create a simple and economical method that makes it possible to take advantage of the production of biomass of microalgae, including cyanobacteria, whether cultured in clean water and chemical fertilizers or in wastewater treatment processes and/or in treatments of agro-industrial effluents, to obtain extracts or concentrates of biofertilizers and biostimulants for agricultural use.

The applicant is unaware of the existence in the prior art of a method for such purpose having similar features to those of the method that is the subject of the present invention.

### DESCRIPTION OF THE INVENTION

The present invention is established and characterized in the independent claims, while the dependent claims describe additional characteristics thereof.

The invention relates to a method for obtaining concentrates of biofertilizers and biostimulants for agricultural use from biomass of microalgae, including cyanobacteria, which comprises the following steps:
a) Producing a release of cellular content of the microalgae, including cyanobacteria, by rupturing the cells thereof,
b) Hydrolyzing with proteolytic enzymes a protein content and cell wall of microalgae, including cyanobacteria, obtained from releasing the cell content resulting from step a) until producing free amino acids, and
c) Adding nitrogen (N), phosphorus (P) and/or potassium (K) excipients to the product of enzymatic hydrolysis obtained in step b).

This makes it possible to produce a concentrate of biofertilizer and biostimulant for agricultural use, whether in extensive or intensive farming, rich in free amino acids, peptides and phytohormones of natural origin from microalgae, including cyanobacteria, which may have been cultured solely for such purpose in clean water and chemical fertilizers, or retrieved from previous wastewater purification processes and/or in treatments of agro-industrial effluents. In the latter case, the production of biomass of microalgae, including cyanobacteria, resulting from the previous process or treatment is recycled for another purpose, i.e. for obtaining extracts or concentrates of biofertilizers and biostimulants for agricultural use, thereby favoring the environment.

### DETAILED DESCRIPTION OF THE INVENTION

The present invention is a method for obtaining concentrates of biofertilizers and biostimulants for agricultural use from biomass of microalgae, including cyanobacteria.

The method starts by producing the release of cellular content from microalgae, including cyanobacteria, by rupturing the cells thereof, which involves the harvesting, concentration and physical homogenization of the biomass of microalgae, including cyanobacteria.

Preferably, the process for rupturing the cells of the microalgae, including cyanobacteria, starts with harvesting approximately 5% to 60% of the volume of a reactor (photobioreactor) containing a variable concentration of between 0.1 g/L and 20 g/L of biomass of microalgae, including cyanobacteria, in suspension.

The biomass of microalgae, including cyanobacteria, to be harvested can be biomass harvested from a culture resulting from wastewater purification processes and/or treatments of agro-industrial effluents, or from animal slurry treatments, organic waste digestates, leachates or agricultural waste, or cultured in clean water and chemical fertilizers for such purpose, i.e. to produce biofertilizers or biostimulants for agricultural use.

Next, the previously harvested biomass of microalgae, including cyanobacteria, is concentrated by decantation, for example, in a decanter medium whose output reaches a solids concentration of biomass of microalgae, including cyanobacteria, of between 10 g/L and 30 g/L. Depending on whether it is required by the microalgae or cyanobacteria, to facilitate sedimentation, a flocculating agent is added, with a concentration of between 0.1% and 20%.

Next, the biomass of microalgae, including cyanobacteria, previously concentrated in the decanter medium is pumped into centrifugal means, for example, a continuous centrifuge, where it is centrifuged until said biomass of microalgae, including cyanobacteria, reaches a solids concentration of between 1% and 20% in the outflow.

Subsequently, the centrifuged biomass of microalgae, including cyanobacteria, is pumped into high-pressure homogenization means, for example, between 100 bar and 1,000 bar, where it is homogenized until ultimately rupturing the cells of said microalgae, including cyanobacteria, thereby releasing the cellular content (metabolites and biomolecules) which is then treated to obtain the phytostimulating agents in the concentrate from which the corresponding biofertilizer and biostimulant for agricultural use will be formulated.

Once the cellular content of the microalgae, including cyanobacteria, is released, the protein content and cell wall of said microalgae, including cyanobacteria, is hydrolyzed with proteolytic enzymes that finish breaking down the cell walls until producing the free amino acids required in the formulation of the biofertilizer and biostimulant; likewise, enzymatic hydrolysis of cell wall debris from cell rupture occurs due to the previous high-pressure homogenization performed.

Preferably, the enzymatic hydrolysis of the protein content and the cell wall of the microalgae, including cyanobacteria, is carried out in a stirred tank-type reactor at controlled pH and temperature. For example, it is preferred to maintain pH between 3.0 and 12.0, temperature between 25°C and 60°C, and stirring at between 50 rpm and 500 rpm.

Also, in order to carry out the enzymatic hydrolysis, a cellulase-type hydrolytic enzyme is firstly added with a concentration of between 0.01% and 20%. The cellulase-type hydrolytic enzyme is responsible for finishing breaking down the cell walls and releasing all cellular content into the reaction medium. It is left to react for approximately between 5 minutes and 600 minutes, at the optimum pH and temperature values of the hydrolytic enzyme used, under constant stirring at between 50 rpm and 500 rpm. During this time, the hydrolysis and rupture of the cellulose that forms the cell wall of the photosynthetic microorganisms that make up the microalgae, including cyanobacteria, which were not previously ruptured during the high-pressure homogenization take place.

Next, once the minutes of reaction with the cellulase enzymes have elapsed, a protease mixture or cocktail consisting of exopeptidase and endopeptidase is added in a ratio of 1:3. This proteolytic reaction is intended to release free amino acids from the constituent protein of the biomass of microalgae, including cyanobacteria, and is allowed to react for approximately between 0.5 hours and 12.0 hours under constant stirring at between 50 rpm and 500 rpm and at optimum temperature and pH values for the enzyme cocktail.

Subsequently, nitrogen (N), phosphorus (P) and/or potassium (K) excipients are added to the previously obtained product of enzymatic hydrolysis, preferably in corresponding ratios that adjust the pH of said product of enzymatic hydrolysis to between 3.0 and 12.0. For example, the excipients added could be inorganic, solid or liquid compounds, a source of nitrogen (N), phosphorus (P) or potassium (K), or nitric acid (HNO₃) and phosphoric acid (H₃PO₄).

Nitrogen (N), phosphorus (P) and/or potassium (K) excipients are added to the product of enzymatic hydrolysis to stabilize said product in order avoid unwanted fermentation once the biofertilizer and biostimulant is packaged in its final container.
Likewise, the product of enzymatic hydrolysis enriched with nitrogen (N), phosphorus (P) and/or potassium (K) excipients (adjusted and stabilized by pH) is preferably packaged aseptically, thereby improving its preservation until use in the preparation of a biofertilizer and biostimulant for agricultural use.

### Example of embodiment

Starting with an urban wastewater purification system, based on a raceway-type photobioreactor with a surface area of 100 m², wherein 30% of its volume, corresponding to 6,000 L, is harvested, with a concentration of 1 g/L of biomass of the microalgae (including cyanobacteria)-bacteria consortium established during the purification process in the photobioreactor.

Next, the volume harvested, with a flow rate of 2 m³/h, is passed through a 1 m³ decanter, collecting a concentrate of biomass of microalgae, including cyanobacteria, of between 2 and 3 g/L. The supernatant is recycled to the photobioreactor, while the concentrated stream of microalgae, including cyanobacteria, is pumped with a flow rate of 0.1 m³/h into a continuous centrifuge where it is centrifuged at 2,300 rpm to a solids concentration of between 25 g/L and 40 g/L.

Subsequently, in the same way as at the outlet of the decanter, the clarified supernatant is recirculated to the photobioreactor, while the centrifuged outflow concentrated in biomass of microalgae, including cyanobacteria, is pumped into a high-pressure homogenizer, where the cells of the microalgae, including cyanobacteria, are ruptured ,with a flow rate of 0.1 m³/h.

The sludge resulting from the process for homogenizing biomass of microalgae, including cyanobacteria, is pumped into a stirred tank-type reactor, with a total volume of 800 L, with temperature and pH control under constant stirring at 50 rpm.

Once the stirred tank-type reactor is filled to 600 L of its volume, 5.6 kg of cellulase are added and the temperature is adjusted to 35°C, maintaining pH between 10 and 12 by constantly adjusting with 35% nitric acid (HNO₃) and a 1.0 molar solution of sodium hydroxide (1M NaOH), for 280 minutes.

After 280 minutes, the protease cocktail is added with a concentration of 0.02%, i.e., 0.12 kg of the 1:3 ratio of exopeptidase and endopeptidase, allowing an additional 9 hours of reaction to elapse at the previously adjusted pH and temperature values.

After the 9 hours of reaction have elapsed, the pH of the reaction product is adjusted to 12.0 by adding nitric acid (HNO₃) and phosphoric acid (H₃PO₄).

Next, the reaction product with adjusted pH and with concentrations of nitrogen (N), phosphorus (P) and/or potassium (K) is pumped to be aseptically packaged in 50 L bottles.

## Claims

1. A method for obtaining concentrates of biofertilizers and biostimulants for agricultural use from biomass of microalgae, including cyanobacteria, which comprises the following stages:
a) Producing a release of cellular content of the microalgae, including cyanobacteria, by rupturing the cells thereof,
b) Hydrolyzing with proteolytic enzymes a protein content and cell wall of microalgae, including cyanobacteria, obtained by releasing the resulting cell content in step a) until producing free amino acids,
c) Adding nitrogen (N), phosphorus (P) and/or potassium (K) excipients to the product of enzymatic hydrolysis obtained in step b).

2. The method according to claim 1, wherein step a) includes the following substeps:
a.1) Harvesting approximately 5% to 60% of a reactor volume containing a variable concentration of between 0.1 g/L and 20 g/L of biomass of microalgae, including cyanobacteria, in suspension,
a.2) Concentrating the biomass of microalgae, including cyanobacteria, harvested in substep a.1) by decantation, reaching a solids concentration of biomass of microalgae, including cyanobacteria, of between 10 g/L and 30 g/L ,
a.3) Centrifuging the biomass of microalgae, including cyanobacteria, concentrated in substep a.2), with the biomass of microalgae, including cyanobacteria, reaching a solids concentration of between 1% and 20%,
a.4) Homogenizing the biomass of microalgae, including cyanobacteria, centrifuged in substep a.3) at high pressure until causing the cell rupture of said microalgae, including cyanobacteria.

3. The method according to claim 2, wherein a flocculating agent is added in substep a.2) to facilitate sedimentation of the biomass of microalgae, including cyanobacteria.

4. The method according to claim 3, wherein the flocculating agent is added in a concentration of between 0.1% and 20%.

5. The method according to claim 1, wherein step b) is carried out in a stirred tank-type reactor at controlled pH and temperature, and includes the following substeps:
b.1) Adding a cellulose-type hydrolytic enzyme in a concentration of between 0.01% and 20%, and allowing it to react for approximately 5 minutes to 600 minutes,
b.2) Adding a mixture of proteases consisting of exopeptidase and endopeptidase in a ratio of 1:3 and allowing it to react for approximately 0.5 to 12.0 hours.

6. The method according to claim 5, wherein the pH is maintained between 3.0 and 12.0, the temperature is maintained between 25°C and 60°C, and stirring is carried out at between 50 rpm and 500 rpm.

7. The method according to claim 1, wherein, in step c), the nitrogen (N), phosphorus (P) and/or potassium (K) excipients are added in such ratios as to adjust the pH of the product of enzymatic hydrolysis to between 3.0 and 12.0.

8. The method according to claim 1 or 7, wherein the excipients added in step c) are solid or liquid inorganic compounds, source of nitrogen (N), phosphorus (P) or potassium (K).

9. The method according to claim 8, wherein the pasteurized product of enzymatic hydrolysis is aseptically packaged.

10. The method according to claim 1, wherein the biomass of microalgae, including cyanobacteria, is cultured in wastewater treatment processes and/or agro-industrial effluent treatments.

11. The method according to claim 1, wherein the biomass of microalgae, including cyanobacteria, is cultured in treatments of animal slurry, organic waste digestates, leachates or agricultural waste.

12. The method according to claim 1, wherein the biomass of microalgae, including cyanobacteria, is cultured in clean water and chemical fertilizers.

13. The method according to claim 1, wherein the biomass to be treated is a microalgae (including cyanobacteria)-bacteria consortium.
